# EUROPEAN PATENT APPLICATION

(11) **EP 2 869 228 A1**
(43) Date of publication of application: **06.05.2015**
(21) Application number: 14189894.0
(22) Date of filing: 22.10.2014
(51) Int. Cl.: G06F 19/00

(54) **Biological information monitor apparatus and central monitor**

(30) Priority: 29.10.2013 JP 2013224642
(71) Applicant: Nihon Kohden Corporation, Shinjuku-ku Tokyo (JP)
(72) Inventor: Wake, Shingo, Tokyo (JP); Yasumaru, Nobuyuki, Tokyo (JP); Ogino, Yoshihiro, Tokyo (JP)
(74) Representative: Brevalex

(57) **Abstract**

A biological information monitor apparatus (1) includes a display unit (6), a measurement unit (2) that measures biological information of a subject, a time measuring unit (3) that measures elapsed time from predetermined time, a recording unit (4) that records an event list including a plurality of events relating to a condition of the subject, and a control unit (7) that controls operations of the respective units. The display unit (6) includes a first area (11) to display the biological information measured by the measurement unit (2), a second area (12) to display the plurality of events and a third area (13) to display the elapsed time. The control unit (7) deletes an event implementation of which has been completed from the second area (12) and then displays an event which is not displayed in the second area (12) until then, in the second area (12).

## Description

### BACKGROUND

The invention relates to a biological information monitor apparatus configured to measure biological information of a subject and to display the measured biological information on a display unit, and a central monitor.

In the related art, a health professional carries out a medical procedure for a subject in accordance with a guideline defined by institutes and hospitals. According to the guideline, when it is diagnosed that a disease of the subject is septicemia, it is required to carry out a rapid and correct medical procedure for the subject.

In the meantime, the health professional perceives a condition of the subject by monitoring a biological signal of the subject with a biological information monitor apparatus. For example, JP-A-2011-98189A discloses a biological information monitor apparatus configured to measure biological information from the subject and to display the biological information on a display unit.

In the guideline in which the medical procedures for the septicemia subject are defined, a plurality of procedures to be carried out in time-series order is defined over a long period of time. For this reason, the health professional should record whether each medical procedure is appropriately carried out in accordance with the guideline and then carry out a next medical procedure while perceiving a condition of the subject through the biological information displayed on a display screen of the biological information monitor apparatus. Therefore, the health professional has a big burden as regards the medical procedure for the subject.

It is therefore an object of the invention to provide a biological information monitor apparatus and a central monitor capable of reducing a burden on a health professional as regards a medical procedure for a subject.

### SUMMARY

(1) According to an aspect of the invention, a biological information monitor apparatus includes a display unit, a measurement unit that measures biological information of a subject, a time measuring unit that measures elapsed time from predetermined time, a recording unit that records an event list including a plurality of events relating to a condition of the subject, and a control unit that controls operations of the respective units. The display unit includes a first area configured to display the biological information measured by the measurement unit, a second area configured to display the plurality of events and a third area configured to display the elapsed time. The control unit deletes an event of the plurality of events implementation of which has been completed from the second area and then displays an event of the plurality of events, which is not displayed in the second area until then, in the second area.

According to the above configuration, the biological information measured from the subject, the events relating to the condition of the subject and the elapsed time from the predetermined time are collectively displayed on the display unit of the one biological information monitor apparatus. Therefore, a health professional can check a content of a medical procedure to be carried out next time while perceiving the condition of the subject through the respective information displayed in the respective areas of the display unit of the biological information monitor apparatus. Also, the event implementation of which has been completed is deleted from the second area of the display unit and only events to be carried out are displayed in the second area. Therefore, the health professional can easily check the display contents of the event list and easily find out a specific event to be carried out next time from the displayed events. In this way, a burden on the health professional as regards the medical procedures for the subject is reduced.
(2) In the biological information monitor apparatus according to the configuration (1), the plurality of events includes a biological information related event relating to the biological information being measured by the measurement unit, and the control unit deletes the biological information related event from the second area when a set condition of the biological information related event is satisfied.

According to the above configuration, regarding the event relating to the biological information being measured in real time, a display of the event is automatically deleted from the second area of the display unit when a predetermined condition is satisfied. Therefore, the health professional can easily manage the event list.
(3) In the biological information monitor apparatus according to the configuration (1) or (2), the display unit further includes a fourth area configured to display a trend based on the biological information of the subject measured with the measurement unit.

According to the above configuration, the health professional can check the trends based on the biological information measured from the subject on the fourth area of the display unit. For this reason, since the health professional can check the effect of the medical procedure and the like and more rapidly check the change in the condition of the subject by observing the trends. Therefore, for example, when a plurality of events having the priority rankings of the substantially same level is displayed in the second area, the health professional can easily determine an event (a medical procedure) to be carried out next time while checking the recent trend.
(4) In the biological information monitor apparatus of any one of the configurations (1) to (3), the recording unit records a history of the event of the plurality of events implementation of which has been completed, and the recording unit displays the history of the event on the display unit.

According to the above configuration, since the history of the event implementation of which has been completed is recorded in the recording unit, the history can be read out from the recording unit and can be again displayed on the display unit. Thereby, a third party can check later the history of the event implementation of which has been completed, the content thereof and the like on the display unit, which improves the operability of the medical procedure.
(5) The biological information monitor apparatus of any one of the configurations (1) to (4), further includes a notification unit, and the control unit controls the notification unit to generate an alarm when there is a non-implemented event of the plurality of events implementation of which has not been completed and a scheduled implementation time of the non-implemented event has elapsed.

According to the above configuration, the event that is not carried out even though the scheduled implementation time has elapsed is warned, so that it is possible to call the attention of the health professional.
(6) According to another aspect of the invention, a central monitor connected to the biological information monitor apparatus according to any one the configurations (1) to (5) through a network and configured to intensively manage the biological information monitor apparatus. Information displayed on the biological information monitor apparatus is displayed in the same display aspect as the biological information monitor apparatus through the network.

According to the above configuration, the health professional can check the information of each subject without moving to places at which the respective subjects stay. Thereby, it is possible to improve the operation efficiency of the health professional and to reduce the burden on the health professional.

### BRIEF DESCRIPTION OF THE DRAWINGS

Fig. 1 is a functional block diagram illustrating a configuration of a biological information monitor apparatus according to an illustrative embodiment of the present invention.
Fig. 2 illustrates an example of a screen that is displayed on a display unit of the biological information monitor apparatus.
Fig. 3 illustrates another example of the screen that is displayed on the display unit of the biological information monitor apparatus.
Fig. 4 illustrates another example of the screen that is displayed on the display unit of the biological information monitor apparatus.
Fig. 5 illustrates another example of the screen that is displayed on the display unit of the biological information monitor apparatus.
Fig. 6 illustrates another example of the screen that is displayed on the display unit of the biological information monitor apparatus.
Fig. 7 is a configuration view illustrating a central monitor connected to the biological information monitor apparatus.
Fig. 8 illustrates another example of the screen that is displayed on the display unit of the biological information monitor apparatus.

### DETAILED DESCRIPTION OF EMBODIMENTS

Hereinafter, an embodiment of a biological information monitor apparatus and a central monitor of the present invention will be described in detail with reference to the drawings.

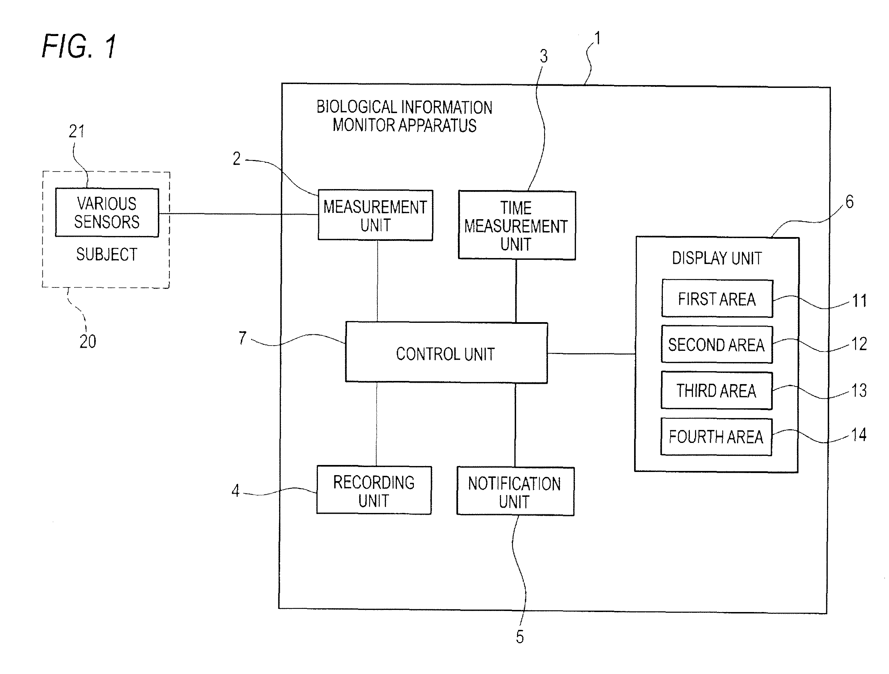

Fig. 1 is a functional block diagram illustrating a configuration of a biological information monitor apparatus 1 according to an illustrative embodiment of the present invention. As shown in Fig. 1, the biological information monitor apparatus 1 can include a measurement unit 2, a time measuring unit 3, a recording unit 4, a notification unit 5, a display unit 6 and a control unit 7 configured to control the respective units.

The biological information monitor apparatus 1 is connected to a variety of sensors 21 including an electrode, a cuff and the like attached to a subject 20, through the measurement unit 2. The biological information monitor apparatus 1 is configured to acquire a plurality of signals relating to the biological information by the connected sensors 21 and to input the acquired signals to the measurement unit 2 of the biological information monitor apparatus 1. The acquired signals may include a heart rate, a blood pressure, a respiration rate, a body temperature, a pulse, a central venous pressure (CVP) and the like.

The measurement unit 2 is configured to measure the biological information of the subject. The measurement unit 2 is configured to acquire measured values relating to a variety of biological information on the basis of the signals input from the sensors 21. The measurement unit 2 is connected in communication with the control unit 7 and is configured to output the acquired measured values of the biological information to the control unit 7.

The time measuring unit 3 consists of a counter configured to measure elapsed time from predetermined time.

The predetermined time means time at which the measurement of the biological information of the subject by the biological information monitor apparatus 1 starts, for example. The elapsed time is measured by counting up a counter value from the time at which the measurement of the biological information starts. In this case, the count up of the counter value is automatically initiated when the measured value relating to the biological information is acquired. The time measuring unit 3 is connected in communication with the control unit 7 and is configured to output the measured elapsed time to the control unit 7.

In the meantime, the predetermined time at which the measurement of the elapsed time starts may be time at which a health professional starts to carry out a medical treatment for a subject. In this case, a start button may be provided so that the health professional can determine the start time of the medical treatment by his or her determination. When the health professional operates the button, the count up of the counter value may be initiated. Also, a configuration whereby it is possible to set the time at which the measurement of the biological information starts or the time at which the health professional starts to carry out a medical treatment as the predetermined time may be provided. Thereby, it is possible to improve the operability of the health professional.

The recording unit 4 is configured to record the information about the subject. The recording unit 4 is configured to record at least an event list, which includes events to be carried out for the subject, and a history of an event, which has been implemented and is thus deleted from the display on the display unit 6. In this embodiment, the event is an item relating to a condition of the subject and corresponds to one item of a check list (a Todo list). For example, there are an event 'culture sample collection-two samples or more' for checking whether two or more types of bloods, which are samples of the culture test, are collected and provided for test, an event 'blood gas measurement' for checking whether a blood gas test is performed, and the like. Also, the event list means a list of events that are prepared and should be carried out for each disease and treatment on the basis of an independent guideline of an institute or hospital. The event list includes a plurality of events relating to the condition of the subject.

When an event is not deleted from the display unit 6 and is still displayed thereon even though scheduled time at which the corresponding event should be carried out has elapsed, the notification unit 5 notifies an alarm by a command from the control unit 7. As a method of notifying the alarm, an audible alarm may be output from a speaker. A display area of the event may be blinked on the display unit 6, together with the audible alarm.

The display unit 6 is provided on a front surface of the biological information monitor apparatus 1 and is configured by a touch panel-type liquid crystal screen. The display unit 6 is connected in communication with the control unit 7 and a display thereof is controlled by a control signal output from the control unit 7. The display unit 6 can display a plurality of display areas, for example, a first area 11, a second area 12, a third area 13 and a fourth area 14, based on a command from the control unit 7.

In the first area 11, the measured value of the biological information acquired by the measurement unit 2 is displayed. The measured value of the biological information is displayed in aspects of a waveform and a numerical value. In the second area 12, at least one of the plurality of events to be carried out as regards the condition of the subject is displayed. In the third area 13, the elapsed time from the predetermined time, which is measured by the time measuring unit 3, is displayed. In the fourth area 14, a trend of the biological information of the subject measured by the measurement unit 2 and a history of the event corresponding to a display of the trend are displayed. The trend of the biological information can be displayed on the basis of the past measured biological information and the biological information being measured in real time.

The control unit 7 is an arithmetic processing circuit including an arithmetic element such as a CPU. In the biological information monitor apparatus 1, the operations of the respective units can be controlled by an operation of hardware such as a circuit element arranged on a substrate of the control unit 7 and the like, an operation of software such as a program stored in the arithmetic element or a combination of the operations.

Subsequently, operations of the biological information monitor apparatus 1 are described which are performed when the health professional carries out a medical procedure for a septicemia subject.

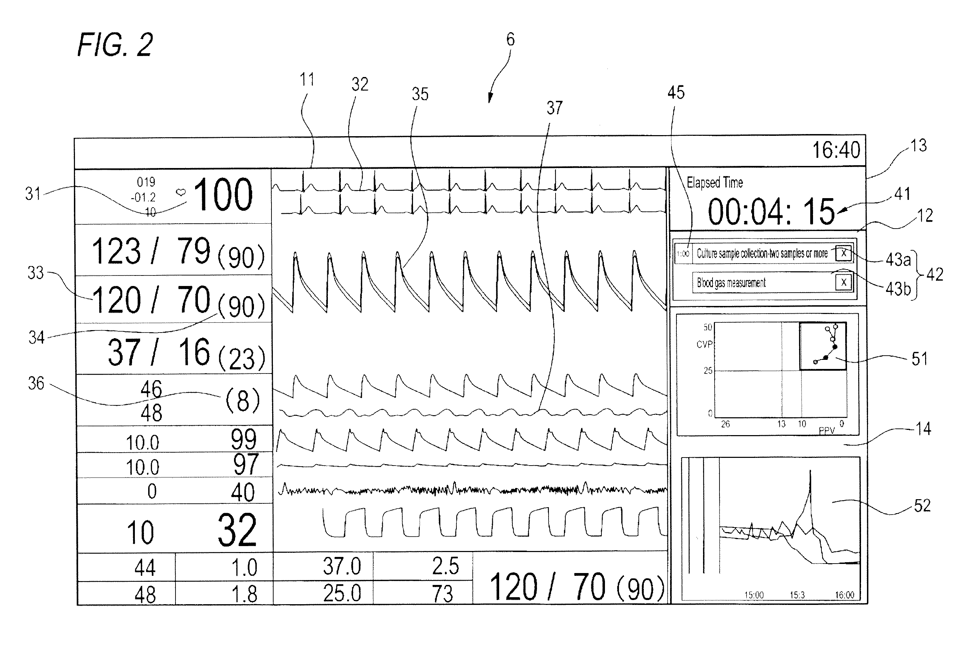

As shown in Fig. 2, when the biological information monitor apparatus 1 starts, the control unit 7 displays the first area 11 in a left 3/4 area and aligns and displays the second area 12, the third area 13 and the fourth area 14 in a right 1/4 area in a vertical direction, when seen from a front surface of the display unit 6. Further, the control unit displays the third area 13 in an upper 1/6 area, the second area 12 in a 1/6 area below the third area 12 and the fourth area 14 in a lower 2/3 area.

When the measurement of the biological information is initiated, a heart rate 31, an electrocardiogram 32, a blood pressure value 33, a mean blood pressure value 34, a blood pressure waveform 35, a CVP value 36, a CVP waveform 37 and the like relating to the biological information measured by the measurement unit 2 are displayed in real time in the first area 11.

Also, a part of an event list 42 relating to the septicemia, which is a disease of the subject, is displayed in the second area 12. The two events, i.e., the event 'culture sample collection-two samples or more' 43a and the event 'blood gas measurement' 43b are displayed in Fig. 2. The event 'culture sample collection-two samples or more' 43a is a check item for checking whether two or more types of bloods, which are samples of the culture test performed so as to find out an infectious cause of the septicemia, are collected and provided for test. The event 'blood gas measurement' 43b is a check item for checking whether a blood gas test is carried out.

Time 45 of '1:00' displayed at the left side of the event 43a indicates scheduled time at which the event should be carried out. In this case, the time indicates that the event 43a should be carried out before the time measurement displayed at elapsed time 41 runs beyond one hour. Also, the event 43b for which the time 45 is not displayed at the left side indicates that the scheduled time thereof to be carried out is not limited.

Also, the elapsed time 41 after the measurement of the biological information starts is displayed in the third area 13. A numerical value of the elapsed time 41 shown in Fig. 2 indicates that 4 minutes and 15 second elapses after the measurement of the biological information of the subject starts. The elapsed time 41 is counted up from zero (00:00:00) at which the measurement of the biological information starts.

Also, the trends of the biological information such as a graph 51 illustrating a relation between past and real time PPV and CVP, a graph 52 illustrating waveform history of the plurality of measured biological information, and the like are displayed in the fourth area 14.

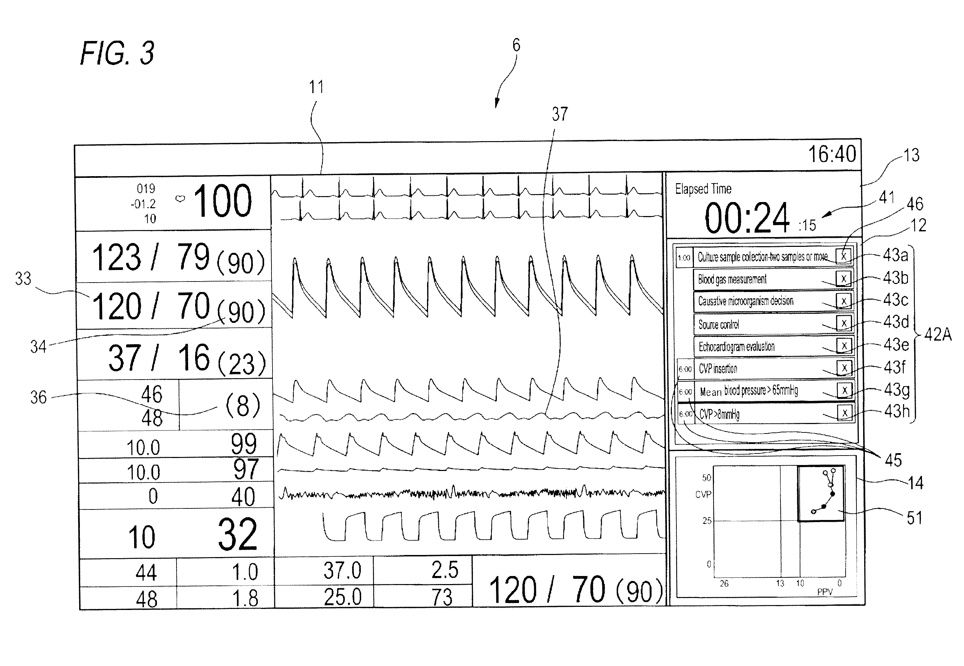

After the measurement of the biological information starts, the health professional can enlarge and display a display size of the second area 12, for example. When the health professional performs a predetermined operation, the control unit 7 displays the second area in a half area of the right 1/4 area of the display unit 6, as shown in Fig. 3, for example.

When the display size of the second area 12 is changed to a size shown in Fig. 3, the number of events to be displayed in the second area 12 is increased, as compared to the number of events displayed in the second area 12 of Fig. 2. In the second area 12 shown in Fig. 2, only two events are displayed. However, in the second area 12 shown in Fig. 3, eight events are displayed. In the second area 12, the events selected in order of the higher priority ranking from the remaining events not displayed until then are sequentially displayed after the already displayed event (here, the event 'blood gas measurement' 43b in contrast with Fig. 2).

That is, in the second area shown in Fig. 3, events of 'causative microorganism decision' 43c, 'source control' 43d, 'echocardiogram evaluation' 43e, 'CVP insertion' 43f, 'mean blood pressure>65 mmHg' 43g and 'CVP>8 mmHg' 43h selected from the remaining events are displayed in an event list 42A. As a result, a total of eight events are displayed with being aligned in descending order from the higher priority ranking to be carried out.

In the meantime, the event 'causative microorganism decision' 43c is a check item for checking whether a causative microorganism of the septicemia can be decided on the basis of a test result of the event 'culture sample collection-two samples or more' 43a. The event 'source control' 43d is a check item for checking whether a treatment of the causative microorganism decided in the event 'causative microorganism decision' 43c starts. The event 'echocardiogram evaluation' 43e is a check item for checking whether an echocardiography is carried out to evaluate a heart function. The event 'CVP insertion' 43f is a check item for checking whether a catheter for measuring a CVP is inserted into the vena cava. The event 'mean blood pressure>65 mmHg' 43g is a check item for checking whether a transfusion management is performed so as to maintain a mean arterial blood pressure value of 65 mmHg or higher. The event 'CVP>8 mmHg' 43h is a check item for checking whether a transfusion management is performed so as to maintain a CVP value of 8 mmHg or higher.

Also, the time 45 of '6:00' displayed at the left sides of the events 43f to 43h indicates that the events 43f to 43h should be carried out before the time measurement displayed at the elapsed time 41 runs beyond six hours. Also, the events 43b to 43e for which the time 45 is not displayed indicates that the priority ranking to be carried out is defined but the scheduled time thereof to be carried out is not limited.

Also, after the measurement of the biological information starts, the health professional can delete the event implementation of which has been completed from the second area 12. For example, when an operator touches and selects a display area of each event displayed in the second area 12, the selected event is deleted from the second area 12. When the event is deleted as described above, an event having a higher priority ranking next to the deleted event is additionally displayed in the second area 12. That is, the event displayed at the lower position of the deleted event is displayed with the order thereof being moved forward. Then, an event having the highest priority ranking of events not displayed until then is additionally displayed at the lowest position of the event list 42A displayed in the second area 12.

Also, when it is determined that an event, which is not necessary to be carried out for the subject, is displayed in the second area 12, the health professional touches a ballot box with X 46 of the event, thereby deleting the corresponding event from the second area.

Also, among the plurality of events displayed in the second area 12 after measurement of the biological information starts, an event relating to the biological information being measured by the sensor 21 through the measurement unit 2 is automatically deleted from the event list 42A when a predetermined setting condition is satisfied. For example, the event 'CVP insertion' 43f, the event 'mean blood pressure>65 mmHg' 43g and the event 'CVP>8 mmHg' 43h of the events displayed in the second area 12 are events relating to the biological information being measured in real time at the measurement unit 2.

The event 'CVP insertion' 43f is first described. When a catheter is inserted into the subject, the measurement of the CVP starts, so that a measured numerical value is displayed at the CVP value 36 in the first area 11 and a measured waveform is displayed at the CVP waveform 37. The health professional sets beforehand a setting condition that a numerical value and a waveform of the CVP are measured (displayed in the first area 11), as a condition of implementation completion of the event 'CVP insertion' 43f, in the biological information monitor apparatus 1. Thereby, when the setting condition is satisfied, it is automatically determined that the implementation of the event is completed, and the display of the event 'CVP insertion' 43f is automatically deleted from the second area 12.

The event 'mean blood pressure>65 mmHg' 43g is also the same. A setting condition that a mean blood pressure value being measured is maintained at 65 mmHg or higher continuously for predetermined time is set in advance as a condition of implementation completion of the event 'mean blood pressure>65 mmHg' 43g. Thereby, when the setting condition is satisfied, it is automatically determined that the implementation of the event is completed, and the display of the event 'mean blood pressure>65 mmHg' 43g is automatically deleted from the second area 12.

The event 'CVP>8 mmHg' 43h is also the same. A setting condition that a CVP value being measured is maintained at 8 mmHg or higher continuously for predetermined time is set in advance as a condition of implementation completion of the event 'CVP>8 mmHg' 43h. Thereby, when the setting condition is satisfied, it is automatically determined that the implementation of the event is completed, and the display of the event 'CVP>8 mmHg' 43h is automatically deleted from the second area 12.

The control unit 7 is configured to control the recording unit 4 to record a history of the event deleted manually or automatically from the second area 12 as described above.

Even when the event is automatically deleted as described above, an event having a higher priority ranking next to the deleted event is additionally displayed in the second area 12, like the manual deletion. That is, the event displayed at the lower position of the deleted event is displayed with the order thereof being moved forward. Then, an event having the highest priority ranking of events not displayed until then is additionally displayed at the lowest position of the event list 42A displayed in the second area 12.

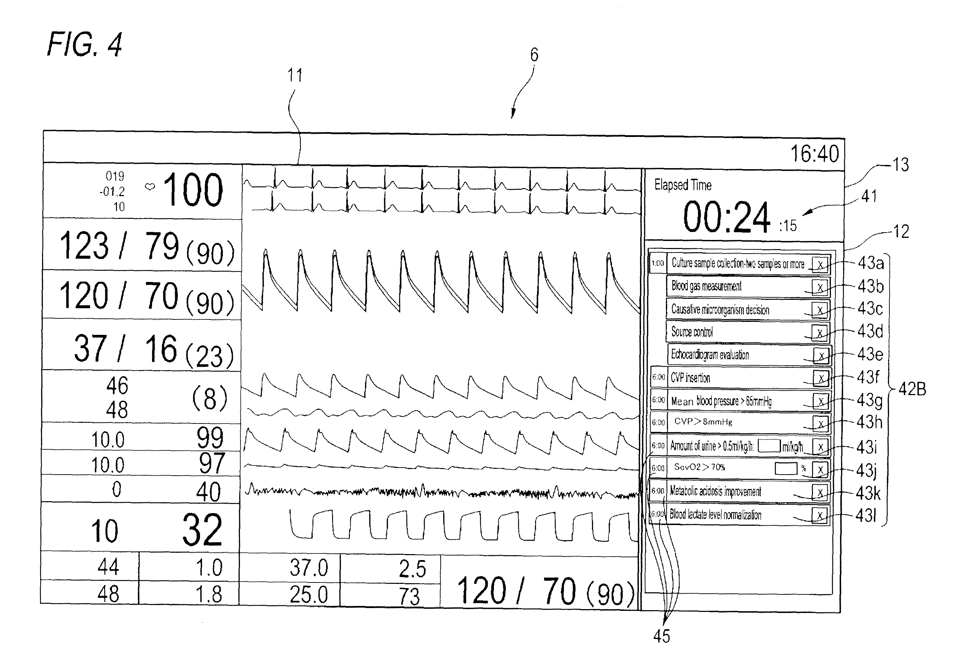

Also, as shown in Fig. 4, the health professional can further enlarge and display the display size of the second area 12. When the health professional performs a predetermined operation, the control unit 7 deletes the fourth area 14 and enlarges the second area 12 in a downward direction in accordance with the operation, as shown in Fig. 4, for example. The number of the events to be displayed in the second area 12 is thus increased. In the second area 12, the events selected in order of the higher priority rankings are sequentially displayed after the already displayed event (here, the event 'CVP>8 mmHg or higher' 43h in contrast with Fig. 3). In an event list 42B shown in the second area 12 of Fig. 4, an event 'amount of urine>0.5 ml/kg/h: □ ml/kg/h' 43i, an event 'ScvO₂>70%: □%' 43j, an event 'metabolic acidosis improvement' 43k and an event 'blood lactate level normalization' 431 are displayed. As a result, a total of 12 events are displayed in the second area 12. The twelve events displayed in the second area 12 are also displayed in descending order from the higher priority ranking to be carried out.

In the meantime, the additionally displayed event 'amount of urine>0.5 ml/kg/h: □ ml/kg/h' 43i is a check item for checking whether an amount of urine per hour for 1 kg of the subject is maintained at 0.5 ml or larger. The event 'ScvO₂>70%: □%' 43j is a check item for checking whether a value of central venous oxygen saturation (ScvO₂) is maintained at 70% or higher. These events are examples of the medical procedure relating to the biological information that cannot be measured with the biological information monitor apparatus 1. In addition, the event 'metabolic acidosis improvement' 43k is a check item for checking whether a management is made to improve an oxidation state of an acid-base balance caused by the metabolism. The event 'blood lactate level normalization' 431 is a check item for checking whether a management is made to improve a blood lactate level and to bring the same close to a normal value.

Also, the time '6:00' displayed at the left sides of the events 43i to 431 indicates that the respective events should be carried out before the time measurement displayed at the elapsed time 41 runs beyond six hours.

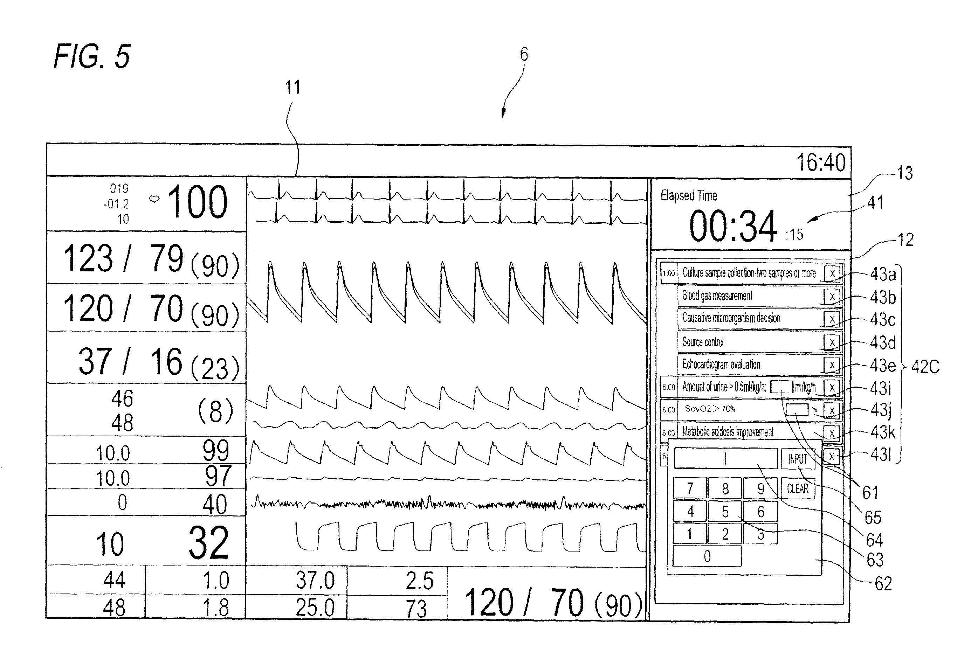

As shown in Fig. 5, the health professional can input measured values into □ parts 61, which are blank parts of the event 'amount of urine>0.5 ml/kg/h: □ ml/kg/h' 43i and the event 'ScvO₂>70%: □%' 43j, from the outside. The measured values of the events are measured by an apparatus different from the biological information monitor apparatus 1 because they cannot be measured with the measurement unit 2 of this embodiment.

When the health professional touches the □ parts 61 in the display areas of the events 43i, 43j, an input screen 62 is displayed with being overlapped over the second area 12. When the health professional inputs a measured value of the measured event with a numerical key 63, the input numerical value is displayed in a display area 64. Then, when the health professional touches an input key 65, the input measured value is decided as a value displayed in the □ part 61 of the event and a series of input operations are completed.

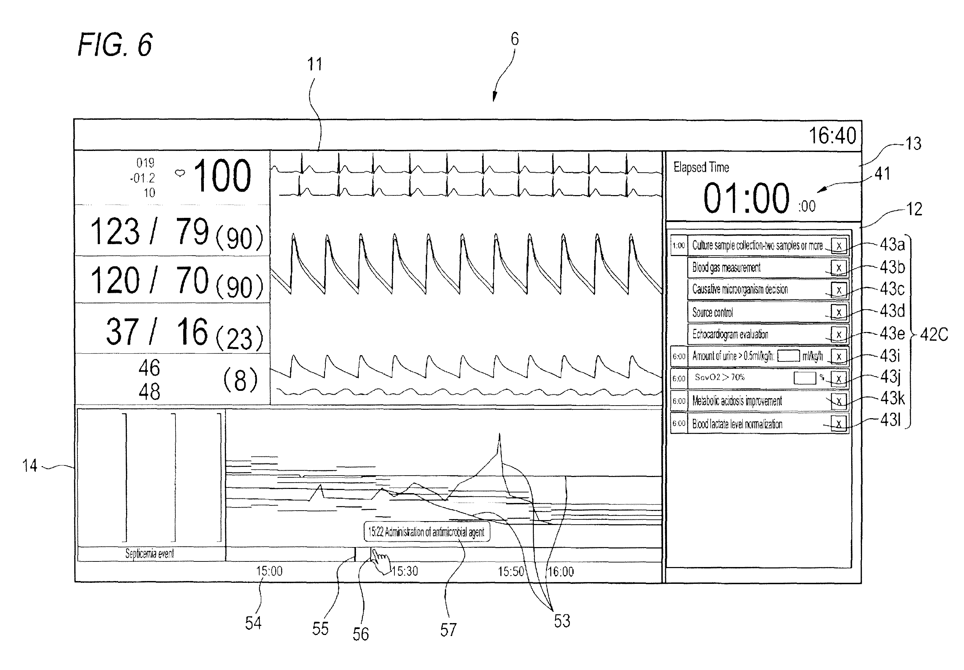

Fig. 6 illustrates the display unit 6 after one hour elapses from the measurement of the biological information of the subject. Regarding the event 'culture sample collection-two samples or more' 43a, the time at which the event should be carried out is set to '1:00'. In this embodiment, even when one hour elapses after the measurement of the biological information starts, the event 'culture sample collection-two samples or more' 43a is not deleted. Therefore, the control unit 7 determines that the implementation of the event 'culture sample collection-two samples or more' 43a is not completed, and blinks the display area of the corresponding event. Further, the notification unit 5 may output an audible alarmfrom the speaker.

Also, as shown in Fig. 6, the health professional may enlarge or modify the display size of the fourth area 14. When the health professional performs a predetermined operation, the control unit 7 reduces the lower side of the first area and enlarges the fourth area 14 to the lower side of the first area 11 in accordance with the operation, as shown in Fig. 6, for example.

In the fourth area 14 of which display size is enlarged, a plurality of waveform history 53 of the biological information measured by the measurement unit 2 is displayed together with measured time 54. Also, the history of the events, which are deleted from the second area 12 because the implementation thereof has been completed, are displayed by vertical bars 55, 56, for example, in correspondence to the display of the waveform history 53. Contents of the history of the events can be displayed by touching the vertical bars. In Fig. 6, an example where the vertical bar 56 is touched is shown. By the touch, a content indicating that implementation of 'dosage of antimicrobial agent' was completed at 15:22 is displayed in a content display unit 57, as a content of the history.

In the related art, while the health professional perceives the condition of the subject by observing the biological information displayed on the display screen of the biological information monitor apparatus, the health professional manages whether each medical procedure could be appropriately carried out in accordance with the guideline, by using a note possessed by the health professional or information stored in a separate system. However, in the guideline in which the medical procedures for the septicemia subject are defined, for example, a plurality of procedures to be carried out in time-series order is defined over a long period of time. For this reason, it is very burdensome to manage all of a progressing status of a medical procedure based on the guideline, the biological information of the subject, the measured time and the like. Also, the health professional may carry out all medical procedures in accordance with the guideline, depending on the experiences of the health professional, and the contents of the medical procedures may be thus different. Also, since it is not possible to unify a check method of checking whether each medical procedure could be appropriately carried out, a post-check by a third person may be difficult.

Also, a managing method by a system configured to collect the information acquired from each subject may be considered. However, it is not possible to display the biological information of the subject in real time during the management by the system. Therefore, since the health professional cannot determine a current condition of the subject only with the display screen of the system, it is difficult for the health professional to rapidly determine a medical procedure to be carried out next time in accordance with the guideline.

In contrast, according to the biological information monitor apparatus 1 of this embodiment, the biological information measured from the subject, the events to be carried out for the subject and the elapsed time for managing the implementation time of the events are displayed on the display unit 6 of the one biological information monitor apparatus 1. Therefore, the health professional can check the contents of the medical procedure to be carried out next time while perceiving the condition of the subject through the respective information displayed in the respective areas of the display unit 6 of the biological information monitor apparatus 1.

Also, the event implementation of which has been completed and the event that is not required to be carried out are deleted from the event list displayed in the second area 12 of the display unit 6 automatically or based on the operation of the health professional. For this reason, since only the events to be carried out are displayed in the second area 12, it is possible to easily check the display contents of the event list and to easily find out a specific event to be carried out next time from the displayed events.

Also, in the second area 12 of the display unit 6, the events having a higher priority ranking to be carried out are displayed in order from above. When the event is deleted as described above, an event having a next high priority ranking is additionally displayed in the second area 12. At this time, as the event is deleted, the other events displayed in the second area 12 are displayed with the orders thereof being moved forward and an event having a higher priority ranking to be carried out next time is selected from the remaining events not displayed until then and is then additionally displayed in the second area 12. For this reason, since the event list is always displayed in order from the event having the higher priority ranking, it is possible to correctly and rapidly recognize the events to be carried out.

Also, the event relating to the biological information being measured in real time is automatically deleted from the second area 12 of the display unit 6 when a predetermined condition beforehand set for the event is satisfied. Therefore, the health professional can easily manage the event list.

Also, the health professional can check the trends based on the biological information measured from the subject on the fourth area 14 of the display unit 6. For this reason, since the health professional can recognize the status of the medical procedure for the subject, the effect of the medical procedure and the like, the health professional can perform a rapid diagnosis. Further, since the biological information configuring the trends includes the biological information being measured in real time, too, it is possible to check the recent trend, thereby recognizing a change in the condition of the subject more rapidly. For example, when the plurality of events having the priority rankings of the substantially same level is displayed in the second area 12, the health professional can easily determine an event (a medical procedure) to be carried out next time while checking the recent trend.

Also, since the history of the event deleted from the second area 12 of the display unit 6 is recorded in the recording unit 4, the history information can be read out from the recording unit 4 and can be again displayed on the display unit 6. Thereby, a third party can check later the history of the event implementation of which has been completed, the content thereof and the like on the display unit 6, which improves the operability of the medical procedure.

Also, the event that is not carried out even though the scheduled implementation time has elapsed is warned through the blink display and the like, so that it is possible to call the attention of the health professional.

Also, regarding the event relating to the biological information that cannot be measured with the biological information monitor apparatus 1, the value measured by the external measuring device can be input to a blank of the event displayed in the second area 12. Therefore, the data that cannot be measured with the biological information monitor apparatus 1 can be collectively managed.

Also, since the display size of the fourth area 14 can be enlarged or modified, as required, it is possible to improve the visibility of the waveforms of the biological information. Thereby, it is possible to easily check a relation between the history of the implemented event and the waveform of the biological information.

As described above, it is possible to reduce a burden on the health professional as regards the medical procedure for the subject.

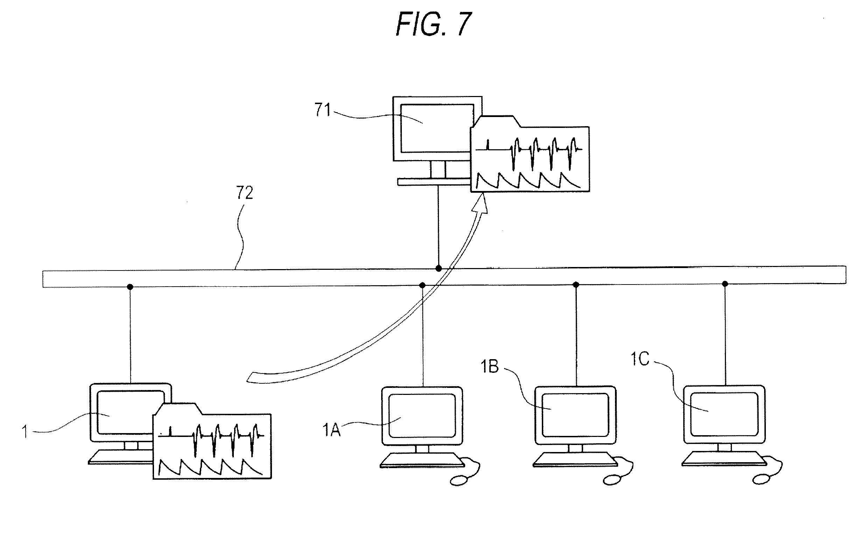

In the below, an example of a central monitor 71 is described with reference to Fig. 7. The central monitor 71 is connected to a plurality of biological information monitor apparatuses 1, 1A, 1B, 1C through a network 72.

The central monitor 71 is a monitor that is provided in a nurse station, in an anteroom for doctors and the like so as to intensively manage the biological information of each subject. All information displayed on the respective biological information monitor apparatuses 1, 1A, 1B, 1C is transmitted to the central monitor 71 through the network 72. Thereby, the central monitor 71 has the substantially same function as the biological information monitor apparatus 1.

Also, the central monitor 71 can display the biological information of each subject measured with the respective biological information monitor apparatuses 1, 1A, 1B, 1C on a display unit of the central monitor 71 in the same display aspects as the respective monitor apparatuses or display the same at the same time.

Also, it is possible to switch the event list of each subject displayed on the display unit to an event list of another subject and to display the same by a simple change operation. For this reason, the health professional can check the information of each subject without moving to the places at which the respective subjects stay. Thereby, it is possible to improve the operation efficiency of the health professional and to reduce the burden on the health professional.

In the meantime, the present invention is not limited to the above embodiment and can be appropriately changed and modified. In addition, the materials, shapes, sizes, the numerical values, the forms, the numbers, the arrangement places and the like of the respective constitutional elements in the above embodiment are arbitrary and are not particularly limited inasmuch as the present invention can be implemented.

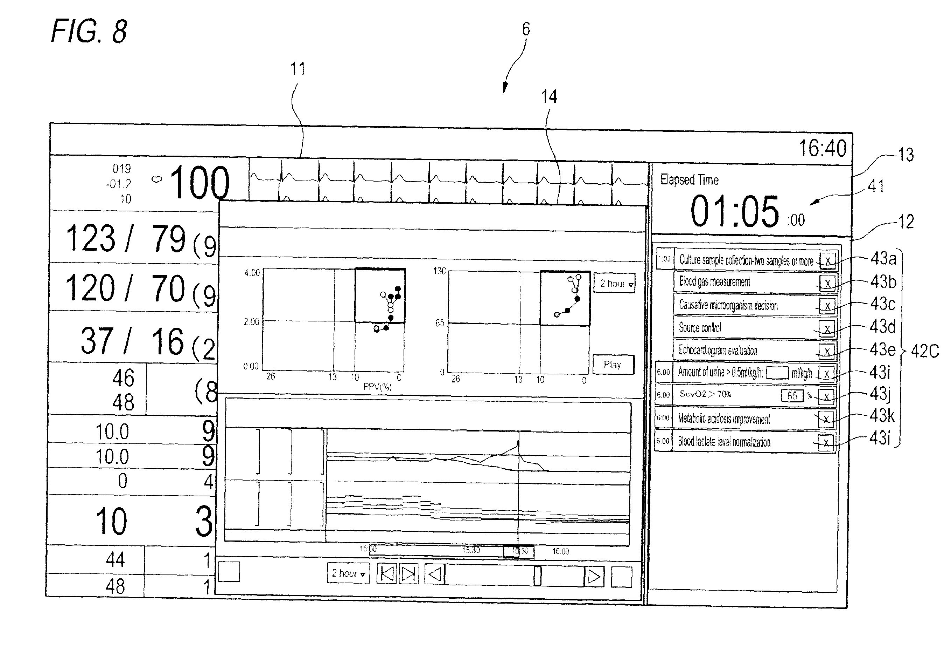

In the above embodiment, the septicemia has been exemplified as the disease of the subject. However, the invention is not limited thereto. For example, the invention can be applied to a determination of brain death, a separation determination of an artificial respirator, and the like. Also, as shown in Fig. 8, the fourth area 14 may be displayed by a popup screen that is displayed with being overlapped over the first area 11.

Also, in Fig. 6, the contents of the history of the event displayed by the touch of the vertical bars 55, 56 and the like may display that the event is deleted as the health professional completes the medical procedure, that the event is deleted because it is determined that it is not necessary to carry out the corresponding event, that the event is automatically deleted, and the like.

## Claims

1. A biological information monitor apparatus (1) comprising:
a display unit (6);
a measurement unit (2) that measures biological information of a subject;
a time measuring unit (3) that measures elapsed time from predetermined time;
a recording unit (4) that records an event list including a plurality of events relating to a condition of the subject; and
a control unit (7) that controls operations of the respective units,
wherein the display unit (6) includes a first area (11) configured to display the biological information measured by the measurement unit (2), a second area (12) configured to display the plurality of events and a third area (13) configured to display the elapsed time, and
wherein the control unit (7) deletes an event of the plurality of events implementation of which has been completed from the second area (12) and then displays an event of the plurality of events, which is not displayed in the second area (12) until then, in the second area (12).

2. The biological information monitor apparatus (1) according to claim 1, wherein the plurality of events includes a biological information related event relating to the biological information being measured by the measurement unit (2), and
wherein the control unit (7) deletes the biological information related event from the second area (12) when a set condition of the biological information related event is satisfied.

3. The biological information monitor apparatus (1) according to claim 1 or 2, wherein the display unit (6) further includes a fourth area (14) configured to display a trend based on the biological information of the subject measured with the measurement unit (2).

4. The biological information monitor apparatus (1) according to any one of claims 1 to 3, wherein the recording unit (4) records a history of the event of the plurality of events implementation of which has been completed, and
the recording unit (4) displays the history of the event on the display unit (6).

5. The biological information monitor apparatus (1) according to any one of claims 1 to 4, further comprising a notification unit (5),
wherein the control unit (7) controls the notification unit (5) to generate an alarm when there is a non-implemented event of the plurality of events implementation of which has not been completed and a scheduled implementation time of the non-implemented event has elapsed.

6. A central monitor (71) connected to the biological information monitor apparatus according to any one of claims 1 to 5 through a network (72) and configured to intensively manage the biological information monitor apparatus (1), wherein information displayed on the biological information monitor apparatus (1) is displayed in the same display aspect as the biological information monitor apparatus (1) through the network.
